Europäisches Patentamt

European Patent Office

Office européen des brevets

(11) Veröffentlichungsnummer: **0 271 668 B1**

# (12) EUROPÄISCHE PATENTSCHRIFT

(45) Veröffentlichungstag der Patentschrift: 18.12.91

(51) Int. Cl.⁵: **A61K  35/32**, A61L 27/00

(21) Anmeldenummer: 87115331.8

(22) Anmeldetag: 20.10.87

Die Akte enthält technische Angaben, die nach dem Eingang der Anmeldung eingereicht wurden und die nicht in dieser Patentschrift enthalten sind.

(54) **Wachstumstimulierendes Material, Herstellungsverfahren und therapeutische Zusammensetzung.**

(30) Priorität: 22.10.86 DE 3635945
22.12.86 DE 3643989

(43) Veröffentlichungstag der Anmeldung:
22.06.88 Patentblatt  88/25

(45) Bekanntmachung des Hinweises auf die Patenterteilung:
18.12.91 Patentblatt  91/51

(84) Benannte Vertragsstaaten:
CH DE FR GB IT LI NL SE

(56) Entgegenhaltungen:
EP-A- 0 121 976          EP-A- 0 128 041
EP-A- 0 148 155          EP-A- 0 182 483
FR-A- 2 564 732          GB-A- 2 176 192
US-A- 4 294 753

PROCEEDINGS OF THE NATIONAL ACADEMY OF SCIENCES OF THE UNITED STATES OF AMERICA, Band 81, Januar 1984, Seiten 371-375, Washington, D.C., US; M.R. URIST et al.: "Purification of bovine bone morphogenetic protein by hydroxyapatite chromatography"

(73) Patentinhaber: Gesellschaft für Biotechnologische Forschung mbH (GBF)
Mascheroder Weg 1
W-3300 Braunschweig-Stöckheim(DE)

(72) Erfinder: Krüger, Wolfgang, Prof. Dr.
Hainbundstrasse 10
W-3400 Göttingen(DE)
Erfinder: Mayer, Hubert, Dr.
Breite Herzogstrasse 24
W-3340 Wolfenbüttel(DE)
Erfinder: Kukoschke, Karl-Gustav
Mascheroder Weg 1
W-3300 Braunschweig(DE)
Erfinder: Delling, Günter, Prof. Dr.
Mascheroder Weg 1
W-3300 Braunschweig(DE)
Erfinder: Schlüter, Klaus-Dieter
Mascheroder Weg 1
W-3300 Braunschweig(DE)

(74) Vertreter: Boeters, Hans Dietrich, Dr. et al
Boeters & Bauer Bereiteranger 15
W-8000 München 90(DE)

**Beschreibung**

Die entzündlichen Erkrankungen des Zahnhalteapparates (Paradontitis, oft auch "Paradontose" genannt) gehören nach Feststellung der WHO zu den am weitesten verbreiteten chronischen Erkrankungen. Durch den dabei auftretenden Abbau des Kieferknochens (vor allem in Form von tiefen Knochentaschen) ist die längerfristige Erhaltung der Zähne gefährdet.

Durch früher propagierte, eher radikale Methoden mit Rekonturierung des Knochens und des Weichgewebes wurde zwar ein gut kontrollierbares Ergebnis erreicht, aber gleichzeitig auch auf die paradontale Regeneration verzichtet und mitunter sogar der Verlust gesunder paradontaler Anteile verursacht; vgl. Schluger 1949 und Ochsenbein 1958.

Ziel der modernen Parodontal-Therapie ist die Regeneration der parodontalen Gewebe durch Säuberung der Zahnwurzeln von mikrobiellen Belägen (Konkrementen) und Entfernung des entzündlich veränderten Gewebes aus den Knochentaschen. Hierfür sind zahlreiche Behandlungsmethoden der paradontalen Defekte entwickelt worden; vgl. u.a. Prichard 1975, Rosling et al. 1976, Polson et al. 1978, Ramfjord & Ash 1979 und Rateitschak et al. 1984 Sowohl nach alleiniger Säuberung des knöchernen Defekts als auch nach Implantation verschiedener Materialien in die Knochentasche soll eine gewisse parodontale Regeneration eintreten; Rosenberg 1971, Schallhorn et al. 1972, Altiere et al. 1979 und Pearlman 1983. Als Implantationsmaterial wird heute vorzugsweise Hydroxylapatit benutzt, der nach klinischen Untersuchungen im Vergleich mit der bloßen Wurzel- und Defektsäuberung eine größere Auffüllung des Defekts zu bewirken scheint; Rabalais et al. 1981, Meffert et al. 1985 und Yukna et al. 1986.

In allen genannten Fällen besteht jedoch ein grundlegendes Problem. Vom oralen Epithel her bilden aussprossende Tochterzellen entlang der Wurzeloberfläche ein neues Epithel (Listgaren 1976), das meistens die gesamte behandelte Wurzeloberfläche überzieht und später sogar apikal des regenerierten Knochens liegen kann; Caton & Nyman 1980, Stahl 1983 und Magnusson et al. 1983. Dieses Epithel kann eine neue Entzündung in der Tiefe des behandelten Knochendefekts begünstigen; Gara et al. 1981. Die grundsätzliche Problematik liegt jedoch darin, daß das Epitheltiefenwachstum schneller abläuft als die knöcherne und desmodontale Regeneration.

Um dieses Epitheltiefenwachstum zu verhindern, wurde nach neueren Arbeiten der Knochendefekt beispielsweise mit Millipore filter abgedeckt, bevor der Mucoperiostlappen reponiert wurde; Nyman et al. 1982, Gottlow et al. 1986 und Magnusson et al. 1985. Dadurch soll die parodontale Regeneration ohne Störung durch das Epitheltiefenwachstum ermöglicht werden. Dieses Verfahren scheint jedoch sehr kompliziert zu sein, wobei die Prognose durch eine hohe Quote von Fehlermöglichkeiten ungewiß ist; Magnusson et al. 1985.

Wachstumsfördernde Aktivitäten aus Knochenmaterial sind von mehreren Arbeitsgruppen beschrieben worden[1, 2].

Die Arbeitsgruppe Baylink et al. isolierte aus humanem Material einen Wuchsstoff mit hohem Molekulargewicht von 83 kD (hSGF = human Sceletal Growth Factor), der das Wachstum von Osteoblast-ähnlichen Zellen aus Hühnerembryonen mit hohem Gehalt an alkalischer Phosphatase förderte[3, 4]. Gleichzeitig wurde das Trockengewicht von embryonalen Hühnerknochen in Organkultur nach Zugabe von hSGF gegenüber unbehandelten Kontrollkulturen gesteigert. In einer späteren Publikation wurden die Zielzellen, die positiv auf diesen Wuchsstoff bzw. humanen Faktor reagieren, umfassender beschrieben. Neben den bereits erwähnten Osteoblast-ähnlichen Zellen wurden auch Haut- und Knorpelzellen vom embryonalen Huhn, humane Knochen- und Hautzellen, humane Osteosarcom-Zellen und Balb/3T3-Zellen als positiv stimulierbar bezeichnet[5].

Ferner wurde die Isolierung eines Wuchsstoffs (bSGF = bovine Skeletal Growth Factor) aus Knochenmaterial des Rindes mit einem Molekulargewicht nach Reduktion mit β-Mercaptoethanol mit einem Molekulargewicht im Bereich von 11 bis 12 kD beschrieben[7]. Die Arbeitsgruppe von Urist et al. isolierte aus Rinderknochen ein Protein mit einem Molekulargewicht von 18,5 ± 0,5 kD, das die Bildung von Knochen in lebenden Tieren induziert. Dieser Wuchsstoff (bBMP = bovine Bone Morphogenetic Protein) wurde in Muskelmaterial lebender Mäuse appliziert[8] und induzierte dort Knochenneubildung; ferner wurden Knochendefekte an Schädelknochen von Hunden nach Applikation von bBMP behandelt.

Ein ähnlicher Wuchsstoff (hBMP = human Bone Morphogenetic Protein) mit einem Molekulargewicht von 17,5 kD wurde von der gleichen Arbeitsgruppe aus humanem Knochenmaterial isoliert[9].

Die Arbeitsgruppe von Seyedin et al.[11] isolierte zwei Proteine aus Rinderknochen, die die Bildung von Knorpelzellen aus Zellen mesodermalen Ursprungs in vitro induzierten (CIF-A und CIF-B = Cartilage Inducing Factor A und B). Das Molekulargewicht von CIF-A und CIF-B wurde mit jeweils 26 kD unter nichtreduzierenden Bedingungen angegeben.

Ferner wurde die Isolierung eines Wuchsstoffes der Ratte (rSGF = rat Sceletal Growth Factor) aus

2

Knochenmaterial der Ratte mit einem Molekulargewicht von 6 bis 17,5 kD beschrieben. Die Arbeitsgruppe von Canalis et al. isolierte zwei Proteine aus dem Kulturmedium von foetalen Rattenschädelknochen in vitro[10], die das Wachstum von Zellen mesodermalen Ursprungs in Organkultur anregten (BDGF-1 und BDGF-2). Die Molekulargewichte dieser Proteine aus dem Kulturmedium wurden mit 10 kD für BDGF-1 und 25 bis 30 kD für BDGF-2 angegeben. Nur BDGF-2 besaß eine mitogene Wirkung.

## Wachstumsstimulierung von Zellen

Der von Baylink et al. isolierte Faktor aus humanem Gewebe (hSGF) bewirkt in erster Linie eine Wachstumsstimulierung von Calvaria-Zellen, die einen hohen Gehalt an alkalischer Phosphatase aufweisen[3, 4].

Die Wirkungen von bBMP sind nicht in Monolayerkulturen getestet worden[8, 9]. CIF-A und CIF-B bewirken eine Wachstumssteigerung von Chondrozyten, jedoch ist diese Wachstumssteigerung nicht in Monolayerkulturen getestet worden[11].

Für BDGF-2 sind die Zielzellen nicht näher bezeichnet worden[5, 10].

## Wachstumsstimulierung von Knochen

hSGF bewirkt eine Zunahme des Trockengewichts von embryonalen Röhrenknochen in Organkultur[3, 4], jedoch ist für hSGF nicht nachgewiesen worden, welche Zellarten an welchen Teilen dieser Organkulturen im Wachstum gefördert werden.

Für bBMP, CIF-A, CIF-B und BDGF-2 wurden keine Ergebnisse veröffentlicht, die Organkulturen embryonaler Röhrenknochen in vitro betreffen.

## Temperaturstabilität der Wachstumsstimulierung

hSGF wird als stabil in einem Temperaturbereich von 55 bis 75 °C beschrieben[3], wobei diese Untersuchungen, die diese Eigenschaft betreffen, an Osteoblast-ähnlichen Zellen durchgeführt wurden[3].

Für bBMP liegen in bezug auf diese Eigenschaft keine Daten vor. CIF-A und CIF-B sind dagegen bei 100 °C 3 min lang stabil[11].

Für BDGF-2 wird Hitzestabilität angegeben[5], jedoch liegen keine Werte vor.

## pH-Stabilität

hSGF ist stabil im Bereich von pH 2,5 bis 10, jedoch wurden keine Zielzellen angegeben, auf denen diese Eigenschaften getestet wurden[3].

Für bBMP und CIF-A und CIF-B liegen keine veröffentlichten Untersuchungen über Säurestabilität vor.

BDGF-2 ist säurestabil[5], ohne daß Angaben über den pH-Bereich und die Zielzellen bekanntgeworden sind.

## Molekulargewichtsbereich

hSGF scheint in zwei Formen vorzuliegen. In 25 mM Phosphat-Puffer (pH 7,2) existiert eine hochmolekulare Form von hSGF[3, 4]. Demgegenüber wird für bSGF nach Behandlung mit 4 M Guanidinium-Hydrochlorid, Umkehrphasen-Hochleistungs-Flüssig-Chromatographie (Reversed Phase HPLC) und Reduktion ein Molekulargewicht von 11 bis 12 kD angegeben[7]. Für die Verwendung von Gelfiltrationssäulen mit 4 M Guanidinium-Hydrochlorid als Fließmittel liegen für hSGF und bSGF keine Angaben vor.

CIF-A und CIF-B haben nach Gelelektrophorese ein apparentes Molekulargewicht von jeweils 26 kD[11].

Über das Molekulargewicht von BDGF-2 bei der Verwendung von Gelfiltrationssäulen mit 4 M Guanidinium-Hydrochlorid als Fließmittel liegen gleichfalls keine Angaben vor.

## Verhalten an Kupfer-II-sulfat-Chelat-Säulen

Über das Verhalten von hSGF, bSGF, bBMP, CIF-A, CIF-B und BDGF-2 liegen keine Angaben über ihr Verhalten an Kupfer-II-Sulfat-Chelat-Säulen vor.

## Verhalten an Umkehrphase-Säulen

bSGF läßt sich an Umkehrphase-Säulen (Reversed Phase HPLC) binden, kann jedoch mit einem Acetonitril-Gradienten wieder eluiert werden[7]; Angaben über den Konzentrationsbereich fehlen. Auch bBMP, CIF-A und CIF-B lassen sich an einer Umkehrphase-Säule binden und mit einem Acetonitril-Gradienten eluieren[8, 11].

Über das Bindungsvermögen von BDGF-2 an Umkehrphase-Säulen liegen keine Angaben vor.

**Gewinnung**

Für die erwähnten Wuchsstoffe des Standes der Technik wurden verschiedene Extraktionsverfahren beschrieben.

Zur Isolation von hSGF verwendete man als Knochenmaterial Enden von humanen Femorae[3]. Es wurde mit EDTA (10 %) demineralisierend extrahiert. Die Art und Weise der Zerkleinerung des Knochenmaterials wurde nicht publiziert.

Für bSGF wird erwähnt, daß ein EDTA-Extrakt aus nicht näher bezeichneten Rinderknochen gewonnen wurde[7]. Über die Art und Weise der Zerkleinerung des Knochenmaterials gibt es keine Angaben. bBMP wurde aus Röhrenknochen gewonnen. Die in flüssigem Stickstoff gekühlten Knochen wurden in einer Whiley-Mühle zu Mehl vermahlen, entfettet und gewaschen. Danach wurde mit 0,6 M HCl demineralisiert[8]. CIF-A und CIF-B wurden aus metatarsalen Rinderknochen gewonnen. Die Knochen wurden in einer durch flüssigen Stickstoff gekühlten Mühle pulverisiert. Die Demineralisierung erfolgte in diesem Fall mit 0,5 M HCl. Extrahiert wurde mit 4 M Guanidinium-Hydrochlorid/10 mM EDTA[11]. BDGF-2 muß nicht extrahiert werden, da dieser Faktor von embryonalem Gewebe in das umgebende Kulturmedium abgegeben wird[10].

rSGF wurde aus nicht näher bezeichneten Röhrenknochen der Ratte mit einer Mischung aus 4 M Guanidinium-Hydrochlorid/EDTA (10 %) extrahiert[6].

**Anreicherung**

Für hSGF sind keine Daten zur Bindung an Hydroxylapatit in der Literatur erwähnt.

Für CIF-A, CIF-B und BDGF-2 gibt es keine Literaturangaben zur Bindung an Hydroxylapatit. bBMP bindet an Hydroxylapatit, ist jedoch weder in Organkultur noch an Monolayer-Kulturen getestet worden. bSGF kann durch Bindung an Hydroxylapatit angereichert werden. Das beschriebene Verfahren besteht aus zwei Stufen:

1) Bindung an Hydroxylapatit, Elution mit Phosphat-Puffer;
2) Bindung an Hydroxylapatit, Elution mit Phosphat-Puffer in Gegenwart von 4 M Guanidinium-Hydrochlorid.

Für die mitogene Gesamtaktivität nach diesen beiden Stufen wird ein Anreicherungsfaktor von etwa 14 bei einer Gesamtausbeute an mitogener Aktivität angegeben, die sich aus den Literaturdaten nicht eindeutig berechnen läßt[7].

rSGF bindet an Hydroxylapatit, jedoch sind keine genauen Bedingungen angegeben worden[6].

EP-A-148 155 beschreibt pharmazeutische Zusammensetzungen, die aus einem osteogenischen Faktor und Kollagen als Trägermaterial bestehen. Bei dem osteogenischen Faktor handelt es sich um ein knochenbildendes Protein vom Schwein.

Demgegenüber ist es Aufgabe der Erfindung, eine therapeutische Zusammensetzung mit einem Wachstum stimulierenden Material und einem festen Träger vorzusehen, das dem Stand der Technik überlegen ist und mit dem insbesondere die Regenerationszeit verkürzt und das Risiko des Epitheltiefenwachstums verhindert wird. Diese Aufgabe wird erfindungsgemäß durch eine therapeutische Zusammensetzung mit einem Wachstum stimulierenden Material und einem festen Träger gelöst, das durch Wachstum stimulierendes Material gekennzeichnet ist, das aus ein oder mehreren wachstumsfördernden (mitogenen) Faktoren besteht oder diese umfaßt und dadurch erhältlich ist, daß man

- entfettetes Knochenmaterial an Metacarpalia und/oder einem Knochengemisch vom Schwein in flüssigem Stickstoff kühlt,
- in einer Presse zu Knochenpulver einer Partikelgröße im Bereich von 0,5 bis 2 mm bei der Temperatur des flüssigen Stickstoffs zerkleinert,
- das anfallende Knochenpulver in an sich bekannter Weise mit der wässerigen Lösung eines Komplexbildners demineralisiert,
- die anfallende wässerige Suspension dialysiert,
- ansäuert und
- erhitzt und
- unlösliche Bestandteile abzentrifugiert und daß man zusätzlich den nach Abzentrifugieren anfallenden wässerigen Überstand

- auf eine Chromatographie-Säule (Hydroxylapatit) gibt und mit Guanidinium-Hydrochlorid (beispielsweise einstufig) eluiert und danach gegebenenfalls
- das Eluat auf eine Gelfiltrationssäule gibt und unter Hochsalzbedingungen (beispielsweise 4 M Guanidinium-Hydrochlorid) im Molekulargewichtsbereiczh von etwa 24 ± 8 kD eluiert, wobei das Wachstum stimulierende Material folgende Merkmale aufweist:

(a) das Wachstum stimulierende Material bewirkt eine dosisabhängige Wachstumsstimulierung von Fibroblasten, Osteoblast-Vorläuferzellen und Chondrozyten aus embryonalem Gewebe des Hühnchens in Monolayer-Zellkulturen;

(b) das Wachstum stimulierende Material bewirkt in vitro eine dosisabhängige Wachstumsstimulierung von embryonalen Röhrenknochen des Hühnchens in Organkultur, wobei es zu einer starken Proliferation von Chondrozyten in der Wachstumszone kommt;

(c) das Wachstum stimulierende Material bewirkt im Mikrotiterplatten-Testsystem keinen gesteigerten Einbau von $^3$H-Thymidin in DNA von Calvaria-Zellen (die histochemisch auf alkalische Phosphatase anfärbbar sind);

(d) die Merkmale (a) und/oder (b) bleiben bei einer Temperatur von 55 bis 75 °C für wenigstens 15 min erhalten;

(e) die Merkmale (a) und/oder (b) bleiben bei einem pH von etwa 3 und etwa 10 für wenigstens 20 min erhalten;

(f) das Wachstum stimulierende Material ist auf einer Gelfiltrationssäule unter Hochsalzbedingungen (beispielsweise etwa 4 H Guanidinium-Hydrochlorid) in einem Molekulargewichtsbereich von 24 ± 8 kD eluierbar;

(g) das Wachstum stimulierende Material läuft durch eine Kupfer-II-sulfat-Chelat-Säule, ohne adsorbiert zu werden, wobei sich die spezifische Aktivität um das 5- bis 20-fache anreichern kann;

(h) das Wachstum stimulierende Material läßt sich bei der Hochleistungs-Flüssig-Chromatographie (HPLC) an einer Umkehrphase-Säule binden und mit einem Acetonitril-Wasser-Gemisch bei einem Acetonitrilgehalt im Bereich von 10 bis 45 % (Volumenbasis) eluieren.

Aufgabe des erfindungsgemäß vorgesehenen Trägers ist es, das Wachstum stimulierende Material zu binden, um ein Ausschwemmen aus dem Applikationsbereich zu verhindern.

Beispiele für erfindungsgemäß verwendbare Träger sind teilweise bis ganz resorbierbare Träger, insbesondere Hydroxylapatit (HAP), Trikalziumphosphat, Kollagen und Kunststoffe. Durch die Resorbierbarkeit des Trägers kann die erfindungsgemäße therapeutische Zusammensetzung im Applikationsbereich schwinden, während sich der Knochen unter der Einwirkung des Wachstum stimulierenden Materials wieder aufbaut.

Bei der erfindungsgemäßen therapeutische Zusammensetzung kann das Verhältnis Wachstum stimulierendes Material : fester Träger = 1:1000 bis 1:200.000 (Volumen:Gewicht) betragen.

Die erfindungsgemäße therapeutische Zusammensetzung kann zur Auffüllung und beschleunigten Heilung von Knochendefekten mit Hohlraumbildung oder für Parodontitis vorgesehen sein.

Eine weitere Aufgabe der Erfindung ist es, ein Wachstum stimulierendes Material für die erfindungsgemäße therapeutische Zusammensetzung vorzusehen.

Diese Aufgabe wird nun durch ein Wachstum stimulierendes Material gelöst, bestehend aus oder umfassend ein oder mehrere wachstumsfördernde (mitogene) Faktoren, das durch die vorstehend angeführten Maßnahmen erhältlich und durch die vorstehend angeführten Merkmale gekennzeichnet ist.

Dabei kann die Anreicherung des Wachstum stimulierenden Materials dadurch überwachbar sein, daß man den Einbau von $^3$H-markiertem Thymidin in die Gesamt-DNA von Osteoblast-Vorläuferzellen und/oder Chondrozyten auf Mikrotiterplatten testet.

Ferner betrifft die Erfindung ein Verfahren zur Gewinnung des Wachstum stimulierenden Materials mit den vorstehend angeführten Maßnahmen. An diese Maßnahmen können sich zusätzlich die Maßnahmen zur Überwachung der Anreicherung anschließen.

## Mikrotiterplatten-Testsystem

Das erfindungsgemäße Wachstum stimulierende Material wird in einem optimierten und in bezug auf die Zellernte automatisierten Testsystem in Mikrotiterplatten getestet. Dieses Testsystem sieht folgende Maßnahmen vor:

(a) Der Einbau von $^3$H-markiertem Thymidin in die Gesamt-DNA von Osteoblasten-Vorläuferzellen wird nach Ausplattieren dieser Osteoblasten-Vorläuferzellen auf Mikrotiterplatten ohne Medienwechsel bei einer Konzentration an foetalem Kälberserum (FCS) durchgeführt, die kleiner als 0,5 % (Volumenbasis) ist.

(b) Die Zelldichte von Osteoblasten-Vorläuferzellen, die mit einer Steigerung des Einbaus von $^3$H-Thymidin in die Gesamt-DNA auf die Zugabe des erfindungsgemäßen Wachstum stimulierenden Materials mit den Wirkungen und Eigenschaften gemäß Anspruch 7 reagieren, liegt im Bereich zwischen 2 500 bis 15 000 Zellen pro Mikrotiterloch (6 mm Durchmesser) im Zeitpunkt des Ausplattierens.

(c) Die maximale Antwort (Einbau von $^3$H-Thymidin in die Gesamt-DNA nach Zugabe des erfindungsgemäßen Wachstum stimulierenden Materials mit den Wirkungen und Eigenschaften gemäß Anspruch 7) wird bei einer Zelldichte von 5 000 Osteoblasten-Vorläuferzellen pro Mikrotiterloch (6 mm Durchmesser) im Zeitpunkt des Ausplattierens und nach einem Wachstum dieser Osteoblasten-Vorläuferzellen von 4 Tagen und bei einer Inkubation von 16 bis 22 h verzeichnet.

(d) Der Einbau von $^3$H-Thymidin in die Gesamt-DNA nach Zugabe des Wachstum stimulierenden Materials gemäß der Erfindung mit den Wirkungen und Eigenschaften gemäß Anspruch 7 wird bei Chondrozyten in Mikrotiterplatten (6 mm Durchmesser pro Mikrotiterloch) bei einer FCS-Konzentration kleiner 0,5 % (Volumenbasis) bei einer Zellkonzentration von 1 250 bis 10 000 Chondrozyten pro Mikrotiterloch im Zeitpunkt des Ausplattierens am ersten, zweiten und dritten Tag nach dem Ausplattieren gesteigert.

(e) Der Einbau von $^3$H-Thymidin in die Gesamt-DNA von adhärenten Zellkulturen in Mikrotiterlöchern wird durch folgende Methode erreicht. Die adhärenten Zellkulturen werden nach Entfernung des radioaktiven Mediums und nach den Waschvorgängen in den Mikrotiterlöchern lysiert. Danach wird die Gesamt-DNA in den Mikrotiterlöchern sauer gefällt. Die gefällte Gesamt-DNA und die in ihr enthaltene Radioaktivität ($^3$H-Thymidin) werden simultan aus mehreren Mikrotiterlöchern mit Hilfe eines automatischen Zellerntegeräts auf Filterpapier überführt. Danach wird die Einbaurate bestimmt.

Nachstehend wird die Erfindung durch Beispiele näher erläutert.

Beispiel 1

50 kg Knochenmaterial vom Schwein wurden bei + 4 °C von Haut, Fleisch und Fett befreit, in flüssigem N$_2$ abgekühlt und bei - 20 °C gelagert. Das Knochenmaterial wurde unter Kühlung mit flüssigem N$_2$ mit der Presseneinrichtung gepreßt, und es ergaben sich 10 kg Gesamtausbeute an Knochenpulver mit einer Partikelgröße von 0,5 bis 2 mm Durchmesser.

1 kg Knochenpulver wurde 2 Wochen in Dialyseschläuchen gegen 10 % EDTA (Gewicht/Volumen) dialysiert und die Dialyselösung zwei Mal gewechselt. Danach wurde die überstehende Lösung in den Dialyseschläuchen zusammen mit dem unlöslichen Rückstand im Starmix homogenisiert. Die Suspension wurde zwei Mal abzentrifugiert, das Pellet jeweils verworfen und der Überstand für 15 min auf 75 °C erhitzt. Das ausfallende Protein wurde durch Zentrifugation entfernt, der Überstand mit 1 N HCl auf pH = 3,0 für 20 min gebracht. Das ausfallende Protein wurde wiederum durch Zentrifugation entfernt, der Überstand mit 1 N NaOH auf pH = 7,2 gebracht. Danach erfolgte Dialyse gegen 25 mM Phosphat-Puffer, pH = 7,2. Es ergaben sich 780 ml Rohextrakt a 218/ug/ml Proteingehalt = 170 mg Gesamtprotein. Dieser Rohextrakt wurde auf eine Hydroxylapatit-Säule mit 5 cm Durchmesser und 30 cm Höhe aufgetragen und mit 25 mM Phosphatpuffer (2 1) und mit 380 ml eines steigenden Gradienten von 25 mM Phosphat bis 100 mM Phosphat gewaschen. Danach erfolgte Elution mit 4 M Guanidinium-HCl. Die aktiven Fraktionen der Elution mit 4 M Guanidinium-HCl wurden gepoolt, gegen 4 M Guanidinium-HCl dialysiert und auf eine Gelfiltrations-Säule mit 4 M Guanidinium-HCl als Fließmittel aufgetragen, die vorher mit Standardproteinen kalibriert worden war. Von dieser Gelfiltrationssäule eluierten die aktiven Fraktionen im Molekulargewichtsbereich von 24 ± 8 kD. Die aktiven Fraktionen der Hydroxylapatit-Säule und der Gelfiltrations-Säule wurden gegen 25 mM Phosphatpuffer dialysiert. Von den einzelnen Reinigungsstufen wurde die dosisabhängige Steigerung des Einbaus von $^3$H-Thymidin in DNA von Osteoblast-Vorläuferzellen bestimmt. Eine Unit wurde definiert als Proteinkonzentration, bei der die halbmaximale Stimulierung dieser Zellen im Einbau von $^3$H- Thymidin stattfindet. Es ergaben sich folgende Werte:

|  | spez. Aktivität (Units/$\mu$g) | Gesamt-Units | Gesamt-Protein ($\mu$g) | Anreicherung |
|---|---|---|---|---|
| Rohextrakt | 13 | 2.210.000 | 170.000 | 1 |
| Hydroxylapatit | 500 | 1.547.000 | 3.094 | 38 |
| Gelfiltration | 3.300 | 330.000 | 100 | 254 |

Beispiele 2 und 3 und Vergleichsbeispiele 1 bis 2

6

Im Tierexperiment wurde beim Hund unter Narkose der Knochen im Unterkieferbereich durch Präparation eines Mucoperiostlappens freigelegt. Danach wurden mit einem normierten innengekühlten Implantatbohrer definierte Bohrlöcher (Durchmesser 2,8 mm, Tiefe 5 mm) angelegt. Diese Bohrlöcher wurden mit einer Zubereitung aus Hydroxylapatit, Wachstum stimulierendem Material gemäß Beispiel 1 oder Kochsalz mit den angegebenen Mengen folgendermaßen gefüllt:

| Beispiel | Zubereitung | | |
|---|---|---|---|
| | HAP (Gew.-teile) | Wachstum stimul. Material (Vol.-teile) | NaCl (Gew.-teile) |
| 2 | $250 \times 10^3$ | 50 | 0 |
| 3 | $250 \times 10^3$ | 50 | 0 |
| Vergleichs-beispiel | | | |
| 1 | $250 \times 10^3$ | 0 | 50 |
| 2 | 0 | 0 | 0 | Bohr-loch bleibt offen |

Nach acht Wochen war das Bohrloch in den Beispielen 2 und 3 sowie im Vergleichsbeispiel 2 vollständig verschlossen, während im Vergleichsbeispiel 1 im oberen Bereich nur eine knöcherne Regeneration mit Ausnahme des zentralen Drittels stattgefunden hatte. Daraus kann man schließen, daß das Wachstum stimulierendes Material bei den Beispielen 2 und 3 eine Verbesserung der Knochenneubildung im Vergleich mit Vergleichsbeispiel 1 bewirkte.

Beispiele 4 und 5 und Vergleichsbeispiele 3 bis 4

Beispiele 2 und 3 und Vergleichsbeispiele 1 und 2 wurden wiederholt, wobei die Auswertung nach drei Wochen erfolgte.

In Beispiel 4 wies das Bohrloch im oberen Bereich und basal bis zur Hälfte (unter Integration der peripheren Zubereitungs-Partikel) eine spongiöse lamelläre Knochenneubildung auf.

Im Beispiel 5 ergab sich im oberen Bereich des Bohrlochs im äußeren Drittel bis zur Hälfte eine lamelläre Knochenneubildung unter Integration der Zubereitungs-Partikel und basal eine fast vollständige Ausfüllung des gesamten Bohrlochs mit Knochenbälkchen unter Integration der Zubereitungs-Partikel.

Im Vergleichsbeispiel 3 war im oberen Bereich des Bohrlochs in der halben Zirkumferenz im äußeren Drittel eine Knochenneubildung zu beobachten, ohne daß die Zubereitungs-Partikel einbezogen waren; basal wurde eine Knochenneubildung in der äußeren Hälfte unter teilweiser Einbeziehung der Zubereitungs-Partikel ermittelt, die zentral bindegewebig eingescheidet waren.

Im Vergleichsbeispiel 4 füllten neugebildete Knochenbälkchen das Bohrloch einer Probe fast vollständig und das Bohrloch einer anderen Probe bis auf das zentrale Drittel aus.

Literaturliste

1.	Simpson E (1984) TIBS 9, pp. 527-530

2. Canalis E (1983) Endocrine Reviews 4, pp.62-77
3. Farley et al. (1982) Biochemistry 21, pp. 3502-3507
4. Farley et al. (1982) Biochemistry 21, pp. 3508-3517
5. Mohan et al. (1984) Calcif Tissue Int 36, pp. 139-145
6. Sibonga et al. (1985) Proc Fed Am Soc Exp Biol 44,p.1099,Nr.4028
7. Jennings et al. (1985) Proc Fed Am Soc Exp Biol 44, p.1099,Nr. 4025
8. Urist et al. (1984) PNAS 81, pp. 371-375
9. Klausner A (1985) Biotechnology 3, pp. 507-511
10. Canalis et al. (1980) Science 210, pp.1021-1023
11. Seyedin et al. (1985) PNAS 82, pp. 2267-2271
12. Puzas et al. (1981) Proc Soc Exper Biol Med 166, pp.113-122

**Patentansprüche**

1. Therapeutische Zusammensetzung mit einem Wachstum stimulierenden Material und einem festen Träger, *gekennzeichnet* durch Wachstum stimulierendes Material, bestehend aus oder umfassend ein oder mehrere wachstumsfördernde (mitogene) Faktoren und dadurch erhältlich, daß man

- entfettetes Knochenmaterial aus Metacarpalia und/oder einem Knochengemisch vom Schwein in flüssigem Stickstoff kühlt,
- in einer Presse zu Knochenpulver einer Partikelgröße im Bereich von 0,5 bis 2 mm bei der Temperatur des flüssigen Stickstoffs zerkleinert,
- das anfallende Knochenpulver in an sich bekannter Weise mit der wässerigen Lösung eines Komplexbildners demineralisiert,
- die anfallende wässerige Suspension dialysiert,
- ansäuert und
- erhitzt und
- unlösliche Bestandteile abzentrifugiert und

daß man zusätzlich den nach Abzentrifugieren anfallenden wässerigen Überstand

- auf eine Chromatographie-Säule (Hydroxylapatit) gibt und mit Guanidinium-Hydrochlorid (beispielsweise einstufig) eluiert und danach gegebenenfalls
- das Eluat auf eine Gelfiltrationssäule gibt und unter Hochsalzbedingungen (beispielsweise 4 M Guanidinium-Hydrochlorid) im Molekulargewichtsbereich von 24 ± 8 kD eluiert, wobei das Wachstum stimulierende Material folgende Merkmale aufweist:

(a) das Wachstum stimulierende Material bewirkt eine dosisabhängige Wachstumsstimulierung von Fibroblasten, Osteoblast-Vorläuferzellen und Chondrozyten aus embryonalem Gewebe des Hühnchens in Monolayer-Zellkulturen;

(b) das Wachstum stimulierende Material bewirkt in vitro eine dosisabhängige Wachstumsstimulierung von embryonalen Röhrenknochen des Hühnchens in Organkultur, wobei es zu einer starken Proliferation von Chondrozyten in der Wachstumszone kommt;

(c) das Wachstum stimulierende Material bewirkt im Mikrotiterplatten-Testsystem keinen gesteigerten Einbau von $^3$H-Thymidin in DNA von Calvaria-Zellen (die histochemisch auf alkalische Phosphatase anfärbbar sind);

(d) die Merkmale (a) und/oder (b) bleiben bei einer Temperatur von 55 bis 75 °C für wenigstens 15 min erhalten;

(e) die Merkmale (a) und/oder (b) bleiben bei einem pH von etwa 3 und etwa 10 für wenigstens 20 min erhalten;

(f) das Wachstum stimulierende Material ist auf einer Gelfiltrationssäule unter Hochsalzbedingungen (beispielsweise etwa 4 M Guanidinium-Hydrochlorid) in einem Molekulargewichtsbereich von 24 ± 8 kD eluierbar;

(g) das Wachstum stimulierende Material läuft durch eine Kupfer-II-sulfat-Chelat-Säule, ohne adsorbiert zu werden, wobei sich die spezifische Aktivität um das 5- bis 20-fache anreichern kann;

(h) das Wachstum stimulierende Material läßt sich bei der Hochleistungs-Flüssig-Chromatographie (HPLC) an einer Umkehrphase-Säule binden und mit einem Acetonitril-Wasser-Gemisch bei einem Acetonitrilgehalt im Bereich von 10 bis 45 % (Volumenbasis) eluieren.

2. Therapeutische Zusammensetzung nach Anspruch 1, *gekennzeichnet* durch einen teilweise bis ganz resorbierbaren Träger, beispielsweise Hydroxylapatit, Trikalziumphosphat, Kollagen oder Kunststoff.

3. Therapeutische Zusammensetzung nach einem der vorhergehenden Ansprüche, *gekennzeichnet* durch ein Verhältnis Wachstum stimulierendes Material : fester Träger = 1:1000 bis 1:200.000 (Volumen:Gewicht).

4. Therapeutische Zusammensetzung nach einem der vorhergehenden Ansprüche zur Auffüllung und beschleunigten Heilung von Knochendefekten mit Hohlraumbildung.

5. Therapeutische Zusammensetzung nach einem der vorhergehenden Ansprüche für Parodontitis.

6. Wachstum stimulierendes Material, bestehend aus oder umfassend ein oder mehrere wachstumsfördernde (mitogene) Faktoren und erhältlich nach den Verfahrensmaßnahmen gemäß Anspruch 1 und mit den Merkmalen gemäß Anspruch 1.

7. Verfahren zur Gewinnung des Wachstum stimulierenden Materials gemäß Anspruch 6, dadurch *gekennzeichnet,* daß man

- entfettetes Knochenmaterial aus Metacarpalia und/oder einem Knochengemisch vom Schwein in flüssigem Stickstoff kühlt,
- in einer Presse zu Knochenpulver einer Partikelgröße im Bereich von 0,5 bis 2 mm bei der Temperatur des flüssigen Stickstoffs zerkleinert,
- das anfallende Knochenpulver in an sich bekannter Weise mit der wässerigen Lösung eines Komplexbildners demineralisiert,
- die anfallende wässerige Suspension dialysiert,
- ansäuert und
- erhitzt und
- unlösliche Bestandteile abzentrifugiert und

daß man zusätzlich den nach Abzentrifugieren anfallenden wässerigen Überstand

- auf eine Chromatographie-Säule (Hydroxylapatit) gibt und mit Guanidinium-Hydrochlorid (beispielsweise einstufig) eluiert und danach gegebenenfalls
- das Eluat auf eine Gelfiltrationssäule gibt und unter Hochsalzbedingungen (beispielsweise 4 M Guanidinium-Hydrochlorid) im Molekulargewichtsbereich von etwa 24 ± 8 kD eluiert.

8. Verfahren nach Anspruch 7, dadurch *gekennzeichnet,* daß man die Anreicherung des Wachstum stimulierenden Materials überwacht, indem man den Einbau von $^3$H-markiertem Thymidin in die Gesamt-DNA von Osteoblast-Vorläuferzellen und/oder Chondrozyten auf Mikrotiterplatten testet.

**Claims**

1. Therapeutic composition with a growth-stimulating material and a solid excipient, characterised by growth-stimulating material consisting of or comprising one or more growth-promoting (mitogenic) factors and obtainable by
   - cooling defatted bone material of metacarpal and/or a bone mixture from pig in liquid nitrogen,
   - comminuting in a press to bone powder of a particle size in the range from 0.5 to 2 mm at the temperature of liquid nitrogen,
   - demineralising the resulting bone powder in a manner known per se with the aqueous solution of a complexing agent,
   - dialysing the resulting agueous suspension,
   - acidifying and
   - heating and
   - removing insoluble constituents by centrifugation and by additionally loading the aqueous supernatant resulting from the centrifugation
   - onto a chromatography column (hydroxyapatite) and eluting with guanidinium hydrochloride (for example in one stage) and then, where appropriate,

- loading the eluate onto a gel filtration column and eluting under high salt conditions (for example 4 M guanidinium hydrochloride) in the molecular weight range of 24 ± 8 kD, where the growth-stimulating material has the following features:

(a) the growth-stimulating material brings about a dose-dependent stimulation of the growth of fibroblasts, osteoblast precursor cells and chondrocytes from embryonic chicken tissue in monolayer cell cultures;

(b) the growth-stimulating material brings about in vitro a dose-dependent stimulation of the growth of embryonic chicken tubular bone in organ culture, there being great proliferation of chondrocytes in the growth zone;

(c) the growth-stimulating material brings about in the microtitre plate assay system no increase in the incorporation of $^3$H-thymidine in DNA of calvarial cells (which can be histochemically stained for alkaline phosphatase);

(d) features (a) and/or (b) are retained for at least 15 min at a temperature of 55 to 75° C;

(e) features (a) and/or (b) are retained for at least 20 min at a pH of about 3 and about 10;

(f) the growth-stimulating material can be eluted on a gel filtration column under high salt conditions (for example about 4 M guanidinium hydrochloride) in a molecular weight range of 24 ± 8 kD;

(g) the growth-stimulating material runs through a copper(II) sulphate chelate column without being adsorbed, it being possible for the specific activity to be concentrated 5- to 20-fold;

(h) the growth-stimulating material can be bound in high performance liquid chromatography (HPLC) to a reverse phase column and eluted with an acetonitrile/water mixture at an acetonitrile content in the range from 10 to 45% (based on volume).

2. Therapeutic composition according to Claim 1, characterised by a partially to entirely absorbable excipient, for example hydroxyapatite, tricalcium phosphate, collagen or synthetic material.

3. Therapeutic composition according to one of the preceding claims, characterised by a growth-stimulating material: solid excipient ratio = 1:1000 to 1:200,000 (volume:weight).

4. Therapeutic composition according to one of the preceding claims for filling in and increasing the rate of healing of bone defects with cavity formation.

5. Therapeutic composition according to one of the preceding claims for periodontitis.

6. Growth-stimulating material, consisting of or comprising one or more growth-promoting (mitogenic) factors and obtainable by the process measures according to Claim 1 and with the features according to Claim 1.

7. Process for obtaining the growth-stimulating material according to Claim 6, characterised in that
   - defatted bone material from metacarpal and/or a bone mixture from pig is cooled in liquid nitrogen,
   - comminuted in a press to bone powder of a particle size in the range from 0.5 to 2 mm at the temperature of liquid nitrogen,
   - the resulting bone powder is demineralised in a manner known per se with the aqueous solution of a complexing agent,
   - the resulting aqueous suspension is dialysed,
   - acidified and
   - heated and
   - insoluble components are removed by centrifugation and
   in that additionally the aqueous supernatant resulting from the centrifugation
   - is loaded onto a chromatography column (hydroxyapatite) and eluted with guanidinium hydrochloride (for example in one stage) and then, where appropriate,
   - the eluate is loaded onto a gel filtration column and eluted under high salt conditions (for example 4 M guanidinium hydrochloride) in the molecular weight range of about 24 ± 8 kD.

8. Process according to Claim 7, characterised in that the concentration of the growth-stimulating material is monitored by assaying the incorporation of $^3$H-labelled thymidine into the total DNA of osteoblast precursor cells and/or chondrocytes on microtitre plates.

**Revendications**

1. Composition thérapeutique comportant un matériau stimulant la croissance et un support solide, caractérisée par un matériau stimulant la croissance constitué d'un ou plusieurs facteurs stimulant la croissance (mitogènes) ou comprenant ce ou ces facteurs, et pouvant être obtenu par le fait que :
   - on refroidit dans de l'azote liquide un matériel osseux dégraissé des métacarpiens et/ou d'un mélange d'os de porc,
   - dans une presse, on broie jusqu'à obtenir une poudre d'os ayant une granulométrie de 0,5 à 2 mm, à la température de l'azote liquide,
   - on déminéralise la poudre d'os ainsi obtenue, d'une manière connue en soi, avec la solution aqueuse d'un complexant,
   - on dialyse la suspension aqueuse obtenue,
   - on acidifie, et
   - on chauffe, et
   - on sépare par centrifugation les composants insolubles, et que
   - on place en outre le surnageant aqueux obtenu après séparation par centrifugation sur une colonne de chromatographie (hydroxylapatite), et on l'élue (par exemple en une étape) avec du chlorhydrate de guanidinium, puis éventuellement,
   - on place l'éluat sur une colonne de filtration sur gel et, dans des conditions fortement salines (par exemple avec du chlorhydrate de guanidinium 4 M), on élue dans l'intervalle de masses moléculaires 24 ± 8 kD le matériau stimulant la croissance ayant les caractéristiques suivantes :
   (a) le matériau stimulant la croissance provoque une stimulation de la croissance, dépendant de la dose, des fibroblastes, des cellules précurseurs d'ostéoblastes et des chondrocytes de tissu d'embryon de poulet en cultures cellulaires monocouches ;
   (b) le matériau stimulant la croissance provoque in vitro une stimulation de la croissance, dépendant de la dose, des os tubulaires d'embryon de poulet en culture d'organe, auquel cas il se produit une forte prolifération de chondrocytes dans la zone de croissance ;
   (c) le matériau stimulant la croissance ne provoque, dans le système d'essai à plaques de microtitrage, aucune augmentation de l'incorporation de thymidine tritiée dans l'ADN de cellules de la calotte crânienne (qui peuvent être colorées par voie histochimique sur la phosphatase alcaline) ;
   (d) les caractéristiques (a) et/ou (b) se conservent pendant au moins 15 minutes à une température de 55 à 75 °C
   (e) les caractéristiques (a) et/ou (b)se conservent pendant au moins 20 minutes à un pH d'environ 3 à environ 10 ;
   (f) le matériau stimulant la croissance peut être élué sur une colonne de filtration sur gel, dans des conditions fortement salines (par exemple du chlorhydrate de guanidinium environ 4 M) sur un intervalle de masses moléculaires de 24 ± 8 kD ;
   (g) le matériau stimulant la croissance traverse une colonne de sulfate de cuivre-II-chélate sans être adsorbé, l'activité spécifique pouvant être multipliée 5 à 20 fois;
   (h) le matériau stimulant la croissance peut se fixer lors d'une chromatographie en phase liquide haute performance (HPLC) sur une colonne à phases inversées et être élué avec un mélange d'acétonitrile et d'eau pour une teneur en acétonitrile de 10 à 45 % (en volume).

2. Composition thérapeutique selon la revendication I, caractérisée par un support partiellement ou totalement résorbable, par exemple en hydroxylapatite, phosphate tricalcique, collagène ou matière plastique.

3. Composition thérapeutique selon l'une des revendications précédentes, caractérisée par un rapport du matériau stimulant la croissance au support solide de 1:1.000 à 1:200.000 (volume:poids).

4. Composition thérapeutique selon l'une des revendications précédentes, pour combler et accélérer la cicatrisation de défauts osseux avec formation de cavités.

5. Composition thérapeutique selon l'une des revendications précédentes, pour la parodontite.

6. Matériau stimulant la croissance constitué d'un ou plusieurs facteurs stimulant la croissance (mitogènes) ou comprenant ce ou ces facteurs, et que l'on peut obtenir par les étapes de procédé

11

selon la revendication I et les caractéristiques de la revendication 1.

7. Procédé pour obtenir le matériau stimulant la croissance selon la revendication 6, caractérisé en ce que:
   - on refroidit dans de l'azote liquide un matériel osseux dégraissé des métacarpiens et/ou d'un mélange d'os de porc,
   - dans une presse, on broie jusqu'à obtenir une poudre d'os ayant une granulométrie de 0,5 à 2 mm, à la température de l'azote liquide,
   - on déminéralise la poudre d'os ainsi obtenue, d'une manière connue en soi, avec la solution aqueuse d'un complexant,
   - on dialyse la suspension aqueuse obtenue,
   - on acidifie, et
   - on chauffe, et
   - on sépare par centrifugation les composants insolubles, et en ce que :
   - on place en outre le surnageant aqueux obtenu après séparation par centrifugation sur une colonne de chromatographie (hydroxylapatite) et on l'élue (par exemple en une étape) avec du chlorhydrate de guanidinium, puis éventuellement,
   - on place l'éluat sur une colonne de filtration sur gel et, dans des conditions fortement salines (par exemple avec du chlorhydrate de guanidinium 4 M), on élue dans l'intervalle de masses moléculaires d'environ 24 ± 8 kD.

8. Procédé selon la revendication 7, caractérisé en ce qu'on surveille l'enrichissement du matériau stimulant la croissance en étudiant sur plaques de microtitrage l'incorporation de thymidine tritiée dans l'ADN total de cellules précurseurs d'ostéoblastes et/ou de chondrocytes.